# EUROPEAN PATENT APPLICATION

(11) **EP 1 834 626 A2**
(43) Date of publication of application: **19.09.2007**
(21) Application number: 07103449.0
(22) Date of filing: 02.03.2007
(51) Int. Cl.: A61K 8/04, A61K 8/73, A61Q 19/08

(54) **Cosmetic or pharmaceutical product for topical use**

(30) Priority: 17.03.2006 IT PD20060092
(71) Applicant: Sala, Stefano, 38100 Trento (IT)
(72) Inventor: Sala, Stefano, 38100 Trento (IT)
(74) Representative: Modiano, Micaela Nadia

(57) **Abstract**

A cosmetic or pharmaceutical product for topical use, comprising, mixed together, the following components:
- propellant, comprising a mixture of butane, propane, isobutane,
- hydroalcohol-based gel,
- at least one active ingredient for topical use.

The components are dispensed on the body surface to be treated from a pressurized vessel which contains them. The propellant evaporates from liquid to gaseous as it exits from the pressurized vessel and upon contact with the body surface to be treated, forming gas bubbles in the gel. The bursting of these bubbles increases the kinetic energy of the gel with the active ingredient, consequently increasing the absorption rate in the layers of the skin.

## Description

The present invention relates to a cosmetic or pharmaceutical product for topical use.

As is known, cosmetic products for topical use differ from pharmaceutical ones depending on the depth of their action within the skin.

When a product for topical use has a high skin penetration power (above a certain threshold value), it is a pharmaceutical product; when the penetration power is lower, it is a cosmetic product (in practice, it has a very localized effect).

Generally, products for topical use, such as creams, ointments, lotions, et cetera, have the problem of the absorption rate of the active ingredient.

In fact, absorption on the part of the skin (more or less deeply) is generally slow, often in contrast with the need to provide a faster cosmetic or curative effect of the active ingredient that is present in the product.

Consider for example the case of analgesic ointments, in which it is important to reduce absorption times in order to alleviate the pain of the user, or the case of anti-cellulite creams, in which the session times to allow absorption of the active ingredient are long and typically require the user to remain motionless for the period of application, with an obvious waste of time.

The aim of the present invention is to provide a cosmetic or pharmaceutical product for topical use which improves the activity of the contained active ingredient.

Within this aim, an object of the present invention is to provide a cosmetic or pharmaceutical product which reduces the absorption times of the contained active ingredient.

Another object of the present invention is to provide a cosmetic or pharmaceutical product for topical use which can be absorbed on a large percentage of the skin area on which it has been distributed.

Another object of the present invention is to provide a cosmetic or pharmaceutical product for topical use which is easy to spread.

This aim and these and other objects, which will become better apparent hereinafter, are achieved by a cosmetic or pharmaceutical product for topical use, characterized in that it comprises, mixed together, the following components:
- propellant, comprising a mixture of butane, propane, isobutane,
- hydroalcohol-based gel,
- at least one active ingredient for topical use,
said components being adapted to be dispensed on the body surface to be treated from a pressurized vessel which contains them, said propellant evaporating from liquid to gaseous as it exits from said pressurized vessel and upon contact with the body surface to be treated, forming gas bubbles in said gel, the bursting of said bubbles increasing the kinetic energy of said gel with said at least one active ingredient, consequently increasing the absorption rate in the layers of the skin.

Advantageously, the cosmetic or pharmaceutical product for topical use comprises, in a percentage on the total weight of the product,
- propellant composed of
   - butane comprised between 25% and 50%,
   - propane comprised between 5% and 10%,
   - isobutane comprised between 5% and 10%,
- hydroalcohol-based gel composed of
   - water comprised between 25% and 50%,
   - denaturated alcohol comprised between 10% and 25%,
   - hydroxy ethyl cellulose comprised between 0.1% and 1.0%,
   - PPG-26-BUTETH-26 comprised between 0.1% and 1.0%,
   - PEG-40-hydrogenated castor oil comprised between 0.1% and 1.0%,
   - POLYQUATERNIUM-4 less than 1.0%.

The product can be used for topical applications in different fields.

Merely by way of example, the product can be used to provide facial products having a moisturizing, emollient, lenitive, protective or anti-wrinkle activity, or to provide body products having a moisturizing, emollient, lenitive, protective, analgesic or anti-cellulite activity.

The product can also be used to provide hair care products having a reinforcing, mineralizing, hair loss-preventing or hair styling activity or foot products with an activity which provides regular perspiration or a deodorant or emollient activity or hand products with emollient and healing activity.

Further characteristics and advantages of the invention will become better apparent from the following detailed description of a preferred but not exclusive embodiment thereof, given hereinafter.

In the exemplary embodiments that follow, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

The product according to the invention is stored within a pressurized vessel provided with a dispenser for distributing the product on the body surface to be treated.

When the product is contained within the vessel, it is constituted substantially by a mixture composed of an active ingredient, hydroalcohol-based gel in which the active ingredient is dissolved, and gaseous propellant, which is in the liquid phase since it is compressed within the vessel.

The gaseous propellant is intended to make the hydroalcohol-based gel exit from the vessel together with the active ingredient and, as explained in greater detail hereinafter, to speed up the absorption of the active ingredient in the skin.

The hydroalcohol-based gel allows to dissolve hydrophilic and hydrophobic active ingredients and is intended to make the mixture viscous and stable.

The active ingredients which can be used can be among the most disparate.

Merely by way of example, if the product can be used for applications of the moisturizing type, it is possible to use active ingredients such as the extract of Aloe vera and glycerin, or cold-pressed vegetable oils, such as oils of jojoba or sweet almond.

Again merely by way of example, if the product can be used for applications of the lenitive and healing type, it is possible to use active ingredients such as extract of Arnica or Hypericum or also "tea tree oil".

If the product can be used for applications of the anti-cellulite type, it is possible to add for example extracts of fucus, theobromine, theophylline, caffeine, genisteine, benzyl or methyl nicotinates.

It is therefore evident that the active ingredient which can be used in the product is a function of the field of use and can therefore be of the most disparate types.

One preferred embodiment of the cosmetic or pharmaceutical product for topical use according to the invention comprises, in a percentage range with respect to the total weight of the product, propellant which is composed of
- butane comprised between 25% and 50%,
- propane comprised between 5% and 10%,
- isobutane comprised between 5% and 10%,
and hydroalcohol-based gel composed of
- water comprised between 25% and 50%,
- denaturated alcohol comprised between 10% and 25%,
- hydroxy ethyl cellulose comprised between 0.1% and 1.0%,
- PPG-26-BUTETH-26 comprises between 0.1% and 1.0%,
- PEG-40-hydrogenated castor oil comprised between 0.1% and 1.0%,
- POLYQUATERNIUM-4 less than 1.0%

The percentage of the active ingredient (or of the active ingredients, since there can be more than one, as needed) must be added to the above.

Practical operation of the product is as follows.

The user dispenses product from the pressurized vessel (an ordinary spray can) onto the body surface to be treated.

The liquid gas (mixture of propane, butane and isobutane) dissolved in the gel evaporates when the product is dispensed, due both to the pressure drop and to the heat of the environment and of the body surface to which it is applied.

The expansion of the gas that accompanies the transition of physical state from liquid to gaseous produces bubbles in the gel.

These gases (which are neither toxic nor pollutant) evaporate and are dispersed into the environment.

In general, part of the gas evaporates directly into the environment and part forms, in the gel deposited on the body surface, bubbles which evaporate due to the heat of the skin and due to the manual massaging action that the user performs after dispensing the product.

Gas evaporation, with the consequent crackling of the bubbles, produces a cooling action due to the removal of heat due to the evaporation of the gas and, as an important characteristic, produces an increase in the kinetic energy of the particles of the gel in which the active ingredient is dissolved.

The removal of heat with corresponding local cooling of the skin produces a feeling of coolness which, in certain applications linked to the treatment of local inflammations, can provide relief.

It has been found experimentally, by means of comparative tests with cream products which contain the same active ingredient but can be applied in conventional manners (therefore without the use of expanding gases but by normal manual application), that the increase in the kinetic energy of the molecules of the active ingredient (or ingredients) contained in the gel due to the gas leads to a significant improvement in the activity of said principle.

In particular, it has been found that said kinetic energy leads to an increase in the skin absorption rate of the active ingredient (with a certain increase in skin absorption depth) and to an increase in the effective area of absorption of the active ingredient with respect to the area of application of the product.

It must be stressed that it is important that the gas mixture, once the product has been applied to the skin, be able to produce said bubbles in the matrix of the gel, since it is their crackling which transmits kinetic energy to the active ingredient; the choice of the three particular gases used is therefore important.

As regards the part of the hydroalcohol-based gel, the various components (except of course for water and alcohol) can be replaced with other equivalents.

For example, the hydroxy ethyl cellulose can be replaced with other components having the same rheologic characteristics, such as for example hydroxy ethyl cellulose, carboxymethyl hydroxy ethyl cellulose, hydroxypropyl methylcellulose or another product having a viscosifying and/or emulsion stabilizing properties.

The PPG-26-BUTETH-26 can be replaced for example with PPG-28-BUTETH-35, PPG-24-BUTETH-27, PPG-20-BUTETH-30 or other products which have anti static and/or emulsifying properties.

The PEG-40-hydrogenated castor oil can be replaced for example with PEG-40-castor oil, PEG-40-hydrogenated castor oil-OIL PCA ISOSTEARATE, PEG-40 HYDROGENATED TALLOW AMINE, or another surfactant and/or product which has an emulsifying property.

The POLYQUATERNIUM-4 can be replaced for example with another type of POLYQUATERNIUM which has antistatic and/or film-forming properties.

In practice it has been found that the invention thus described achieves the intended aim and objects.

In particular, the present invention provides a cosmetic or pharmaceutical product for topical use which allows to increase the absorption rate of the active ingredient (or ingredients) in the skin and to increase the actual absorption area of the active ingredient with respect to the product application area.

This has been achieved by transferring kinetic energy to the active ingredient and to the substances which carry it by means of the phenomenon of the rapid expansion of gas bubbles contained in the gel in which the active ingredient is dissolved.

The invention thus conceived is susceptible of numerous modifications and variations, all of which are within the scope of the appended claims; all the details may further be replaced with other technically equivalent elements.

In practice, the materials employed, so long as they are compatible with the specific use, as well as the dimensions, may be any according to requirements and to the state of the art.

The disclosures in Italian Patent Application No. PD2006A000092 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A cosmetic or pharmaceutical product for topical use, **characterized in that** it comprises, mixed together, the following components:
- propellant, comprising a mixture of butane, propane, isobutane,
- hydroalcohol-based gel,
- at least one active ingredient for topical use,
said components being adapted to be dispensed on the body surface to be treated from a pressurized vessel which contains them, said propellant evaporating from liquid to gaseous as it exits from said pressurized vessel and upon contact with the body surface to be treated, forming gas bubbles in said gel, the bursting of said bubbles increasing the kinetic energy of said gel with said at least one active ingredient, consequently increasing the absorption rate in the layers of the skin.

2. The cosmetic or pharmaceutical product for topical use, according to claim 1, **characterized in that** said propellant comprises, as a percentage on the total weight of the product,
- butane comprised between 25% and 50%,
- propane comprised between 5% and 10%,
- isobutane comprised between 5% and 10%.

3. The cosmetic or pharmaceutical product for topical use according to claim 1 or 2, **characterized in that** said hydroalcohol-based gel comprises, as a percentage on the total weight of the product,
- water comprised between 25% and 50%,
- denaturated alcohol comprised between 10% and 25%.

4. The cosmetic or pharmaceutical product for topical use according to claim 3, **characterized in that** said hydroalcohol-based gel comprises, as a percentage on the total weight of the product,
- at least one element having viscosifying and/or stabilizing properties for emulsions, comprised between 0.1% and 1.0%,
- at least one element which is a surfactant and/or has antistatic and/or emulsifying and/or film-forming properties, comprised between 0.1% and 1.0%.

5. The cosmetic or pharmaceutical product for topical use according to claim 4, **characterized in that** said at least one element having viscosifying and/or stabilizing properties for emulsions is constituted by hydroxy ethyl cellulose or other substance having equivalent properties.

6. The cosmetic or pharmaceutical product for topical use according to claim 4, **characterized in that** it comprises an element having anti static and/or emulsifying properties constituted by PPG-26-BUTETH-26 or another substance having equivalent properties.

7. The cosmetic or pharmaceutical product for topical use according to claim 4, **characterized in that** it comprises an element with antistatic and/or film-forming properties which is constituted by POLYQUATERNIUM or another substance which has equivalent properties.

8. The cosmetic or pharmaceutical product for topical use according to claim 4, **characterized in that** it comprises an element which is a surfactant element and/or has emulsifying properties and is constituted by PEG-40-HYDROGENATED CASTOR OIL or another substance having equivalent properties.

9. The cosmetic or pharmaceutical product for topical use according to one or more of the preceding claims, **characterized in that** it comprises, as percentages on the total weight of the product,
- butane comprised between 25% and 50%,
- propane comprised between 5% and 10%,
- isobutane comprised between 5% and 10%,
- water comprised between 25% and 50%,
- denaturated alcohol comprised between 10% and 25%,
- hydroxy ethyl cellulose comprised between 0.1% and 1.0%,
- PPG-26-BUTETH-26 comprised between 0.1% and 1.0%,
- PEG-40-hydrogenated castor oil comprised between 0.1% and 1.0%,
- POLYQUATERNIUM-4 less than 1.0%.

10. Use of a cosmetic or pharmaceutical product, as set forth in one or more of claims 1-9, for topical application.
